# EUROPEAN PATENT APPLICATION

(11) **EP 4 763 045 A1**
(43) Date of publication of application: **24.06.2026**
(21) Application number: 24220638.1
(22) Date of filing: 17.12.2024
(51) Int. Cl.: A61B 1/045, A61B 1/24, A61B 1/00, A61B 5/00, A61B 34/20

(54) **CONTROLLING IMAGING MODALITY**

(71) Applicant: Koninklijke Philips N.V., 5656 AE Eindhoven (NL)
(72) Inventor: VLUTTERS, Ruud, Eindhoven (NL); GALLUCCI, Alessio, Eindhoven (NL)
(74) Representative: Philips Intellectual Property & Standards

(57) **Abstract**

Proposed concepts thus aim to provide schemes, solutions, concepts, designs, methods and systems pertaining to controlling imaging modality of an oral imaging device. In particular, embodiments aim to provide a method for controlling imaging modality of an oral imaging device by basing the decision to switch imaging modality on the location and/or motion of the camera of the oral imaging device. In other words, it is proposed that by determining a location, velocity, and/or acceleration of the camera of the oral imaging device, the oral imaging device can time its switching of imaging modality more optimally.

## Description

### FIELD OF THE INVENTION

This invention relates to the field of controlling imaging modality of an oral imaging device.

### BACKGROUND OF THE INVENTION

Some oral imaging cameras are capable of capturing images in more than one imaging modality (e.g., white light, quantitative light-induced fluorescence (QLF), infrared light, change of aperture, etc.), and it is useful to capture images of a subject's mouth in multiple imaging modalities, however, switching imaging modalities takes time. Scanning a subject's mouth by switching imaging modality at each tooth such that each tooth can be imaged in multiple imaging modalities can take a significant amount of time due to the significant number of switches that need to be made. This may, in turn, be frustrating for a user.

### SUMMARY OF THE INVENTION

The invention is defined by the claims.

According to examples in accordance with an aspect of the invention, there is provided a computer-implemented method for controlling imaging modality of an oral imaging device, the method comprising: determining a location and/or motion of a camera of the oral imaging device relative to a subject's mouth, wherein motion comprises at least one of: velocity; and acceleration; and controlling the oral imaging device to switch from a first imaging modality to a second imaging modality based on the determined location and/or motion.

Proposed concepts thus aim to provide schemes, solutions, concepts, designs, methods and systems pertaining to controlling imaging modality of an oral imaging device. In particular, embodiments aim to provide a method for controlling imaging modality of an oral imaging device by basing the decision to switch imaging modality on the location and/or motion of the camera of the oral imaging device.

In other words, it is proposed that by determining a location, velocity, and/or acceleration of the camera of the oral imaging device, the oral imaging device can time its switching of imaging modality more optimally. For example, the oral imaging device could be controlled to switch imaging modality while it is moving from one tooth to another such that the user does not have to wait for the oral imaging device to switch once it is at the second tooth. In another example, after one side of a jaw is imaged in a first imaging modality, then it could be determined that the camera has reached the end of that side of the jaw (i.e., has finished its scanning stroke, with its velocity and acceleration now zero, and being located at the subject's molar), and the imaging modality of the oral imaging device could be switched such that on a backstroke (going back over the teeth already imaged), they can be imaged using a second, different imaging modality without the user having to have waited for the oral imaging device to switch imaging modality at each tooth individually.

Furthermore, by basing the switching of the oral imaging device's imaging modality on the location of the camera, prior knowledge about the presence of oral health features can be used (such as whether plaque is present at said location) to, for example, switch to an imaging modality beneficial for imaging the features known to be present at said location.

Ultimately, an improved method for controlling imaging modality of an oral imaging device is provided.

In some embodiments, determining a motion of the camera may comprise determining the motion of the camera based on data from an inertial measurement sensor associated with the oral imaging device. This may provide an effective way to obtain the motion of the camera.

In some embodiments, determining a motion of the camera may comprise capturing a series of images of at least one oral feature of the subject, wherein an oral feature comprises a portion of at least one of: a tooth; gum; tongue; and a lip; and analyzing the captured series of images to determine the motion of the camera. This may allow the motion of the camera to be obtained without the need for additional equipment.

In some embodiments, controlling the oral imaging device may comprise controlling the oral imaging device to switch from a first imaging modality to a second imaging modality when the camera is determined to have stopped moving. For example, the camera typically stops moving when a user has reached the end of a stroke - this may thus be a particularly beneficial time to switch imaging modality so that that the images of the same part of the subject's mouth can be captured in a new imaging modality on a backstroke.

In some embodiments, the method may further comprise predicting a future location of the camera at a future time point based on the determined motion of the camera and a determined present location of the camera; and wherein controlling the oral imaging device comprises controlling the oral imaging device to switch from a first imaging modality to a second imaging modality such that the oral imaging device will be in the second imaging modality at the future time point. In this way, use of the oral imaging device may be made more efficient such that the oral imaging device may essentially pre-emptively switch imaging modality so that it is in the correct imaging modality by the time it reaches a particular location (e.g., the front or back of a user's mouth). A user may then not have to wait once they reach said location for the oral imaging device to switch imaging modality.

In some embodiments, the method may further comprise determining the presence of an oral health feature at the predicted future location of the camera based on the predicted future location and oral health data describing the presence and location of at least one oral health feature in the mouth of the subject; and controlling the oral imaging device based on the determined presence of the oral health feature. In this way, prior knowledge about locations in a subject's mouth can be leveraged such that if it is known that a particular oral health feature is present at a predicted future location of the camera (e.g., plaque), the oral imaging device can be controlled to, for example, put the device in the best available imaging modality for imaging said oral health feature.

In some embodiments, a location of the camera may be determined, and controlling the oral imaging device may comprise determining the presence of an oral health feature in view of the camera at the determined location based on the determined location and oral health data describing the presence and location of at least one oral health feature in the mouth of the subject; and controlling the oral imaging device based on the determined presence of the oral health feature. In this way, prior knowledge about locations in a subject's mouth can be leveraged such that if it is known that a particular oral health feature is present at a current location of the camera, the oral imaging device can be controlled to, for example, put the device in the best available imaging modality for imaging said oral health feature.

In some embodiments, the method may further comprise selecting the second imaging modality from a plurality of available imaging modalities based on the type of oral health feature. In this way, if the oral imaging device has three or more imaging modalities, the most beneficial for the type of oral health feature to-be-imaged can be switched to.

In some embodiments, an oral health feature may comprise at least one of: a cavity; a crown; a bridge; a filling; plaque; tartar; gingivitis; periodontitis; tooth erosion; an abscessed tooth; malocclusion; a denture; a dental implant; braces; an impacted tooth; and a tumor. These may each be useful oral health features to determine the presence of and thus allow a beneficial imaging modality for imaging said feature to be chosen.

In some embodiments, the oral health data may further describe the presence and location of at least one predicted oral health feature in the mouth of the subject. This may thus allow the oral imaging device to be controlled based on oral health features which are not 'known' to be present at a particular location in the subject's mouth but are predicted to be. For example, it may be known that if a subject has plaque on tooth 13, it is highly likely that they will also have plaque on tooth 14. The prediction of there being plaque present on tooth 14 can thus be used to control the oral imaging device (i.e., to select the imaging modality for imaging tooth 14).

In some embodiments, the second imaging modality may comprise at least one of: white light; quantitative light-induced fluorescence; fluorescence imaging with reflectance enhancement; and infrared light. These may be particularly useful imaging modalities for imaging various different oral health features.

In some embodiments, determining a location of the camera may comprise capturing an image of at least one oral feature of the subject, wherein an oral feature comprises a portion of at least one of: a tooth; gum; tongue; and a lip; and analyzing the captured image to determine the location of the camera. In this way, the location of the camera may be determined without the need for any additional component or device.

In some embodiments, determining location and/or motion of the camera may comprise using a machine-learning model trained to determine the relative location and/or motion of the camera relative to a subject's mouth based on data from an inertial measurement sensor associated with the oral imaging device and/or at least one image of at least one oral feature of the subject, wherein an oral feature comprises a portion of at least one of: a tooth; gum; tongue; and a lip. A machine-learning model may provide a particularly effective way to determine the location and/or motion of the camera.

In some embodiments, the method may further comprise sequentially capturing an image of each of at least two different teeth of the subject respectively using the oral imaging device in the first modality. This may enhance the time efficiency of the method, ensuring that the oral imaging device is not needlessly switching imaging modality after imaging only one tooth in the first imaging modality.

According to another aspect of the invention, there is provided a computer program comprising code means for implementing the method of any herein disclosed method when said program is run on a processor arrangement.

According to another aspect of the invention, there is provided a system for controlling imaging modality of an oral imaging device, comprising: a processor arrangement configured to: determine a location and/or motion of a camera of the oral imaging device relative to a subject's mouth, wherein motion comprises at least one of: velocity; and acceleration; and control the oral imaging device to switch from a first imaging modality to a second imaging modality based on the determined location and/or motion.

Thus, there may be proposed concepts for controlling imaging modality of an oral imaging device, and this may be done based on determining the location and/or motion of a camera of the oral imaging device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling imaging modality of an oral imaging device according to a proposed embodiment;
Fig. 2 is a flow diagram of a method for controlling imaging modality of an oral imaging device comprising determining a motion of a camera according to a proposed embodiment;
Fig. 3 is a flow diagram of a method for controlling imaging modality of an oral imaging device comprising determining a location of a camera according to a proposed embodiment;
Fig. 4 is a flow diagram of a method for controlling imaging modality of an oral imaging device comprising determining the presence of an oral health feature according to a proposed embodiment;
Fig. 5 is a flow diagram of a method for controlling imaging modality of an oral imaging device comprising predicting a future location of the camera according to a proposed embodiment;
Fig. 6 is a simplified block diagram of a system for controlling imaging modality of an oral imaging device according to a proposed embodiment; and
Fig. 7 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling imaging modality of an oral imaging device. According to proposed concepts, a number of possible solutions may be implemented separately or jointly. That is, although these possible solutions may be described below separately, two or more of these possible solutions may be implemented in one combination or another.

Embodiments of the invention aim to provide a method for controlling imaging modality of an oral imaging device. This can be achieved by basing the decision to switch imaging modality on the location and/or motion of the camera of the oral imaging device.

In other words, it is proposed that by determining a location, velocity, and/or acceleration of the camera of the oral imaging device, the oral imaging device can time its switching of imaging modality more optimally. For example, the oral imaging device could be controlled to switch imaging modality while it is moving from one tooth to another such that the user does not have to wait for the oral imaging device to switch once it is at the second tooth. In another example, after one side of a jaw is imaged in a first imaging modality, then it could be determined that the camera has reached the end of that side of the jaw (i.e., has finished its scanning stroke, with its velocity and acceleration now zero, and being located at the subject's molar), and the imaging modality of the oral imaging device could be switched such that on a backstroke (going back over the teeth already imaged), they can be imaged using a second, different imaging modality without the user having to have waited for the oral imaging device to switch imaging modality at each tooth individually.

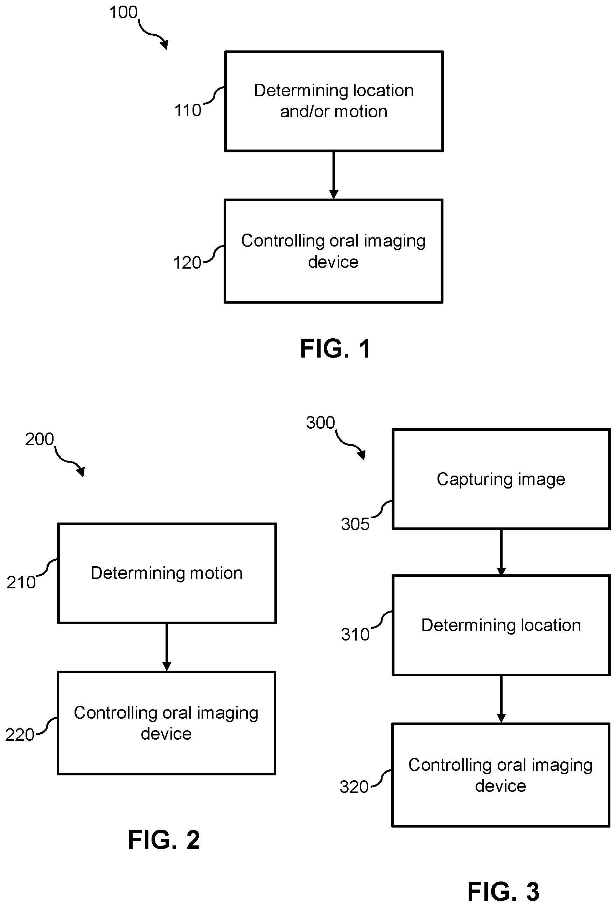

Referring now to Fig. 1, there is depicted a simplified flow diagram of a method 100 for controlling imaging modality of an oral imaging device according to a proposed embodiment.

The method 100 begins with the step 110 of determining a location and/or motion of a camera of the oral imaging device relative to a subject's mouth. Motion comprises at least one of velocity and acceleration, such that step 110 could alternatively be understood as determining at least one of: a location; a velocity; and an acceleration of a camera of the oral imaging device. It should be noted that in some embodiments, the oral imaging device can comprise a plurality of cameras, and thus step 110 would be understood as determining a location and/or motion of at least one of the plurality of cameras of the oral imaging device. It should also be noted that an oral imaging device is to be understood as a device which images a subject's mouth from within their mouth, i.e., intraoral. This does not mean that the entire device must be within the subject's mouth, only at least a camera of the oral imaging device.

In some embodiments, determining location and/or motion of the camera (i.e., step 110) can comprise using a machine-learning model trained to determine the location and/or motion of the camera relative to a subject's mouth based on data from an inertial measurement sensor associated with the oral imaging device and/or at least one image of at least one oral feature of the subject, wherein an oral feature comprises a portion (i.e., part or whole) of at least one of: a tooth; gum; tongue; and a lip. To be clear, an oral feature is a separate (broader) classification of features to an oral health feature. Whereas oral health features are concerned with specific features related to oral health (e.g., plaque or cavities), an oral feature is merely any feature/object within a subject's mouth (e.g., tooth, gum, tongue, lip, etc.) An oral feature may thus also be referred to as an oral object or a mouth feature/object. For instance, a suitable image could be captured of one tooth of the subject, or of two teeth of the subject, or of the tongue of the subject, or a portion of the subject's gum, etc. A machine-learning model provides a particularly effective way to determine the location and/or motion of the camera. As the skilled person would appreciate, in some embodiments, the machine-learning model can comprise a trained artificial neural network. It should also be noted that machine-learning models or artificial neural networks can also be used for other suitable tasks disclosed herein (as would be apparent to the skilled person).

It should be noted that in addition to the inertial measurement sensor (or instead of it) data from other sensors can also be used, for example, a time of flight sensor or a touch/pressure sensor associated with the oral imaging device. The skilled person would, of course, understand how data from these sensors could be used to aid the determination of a location and/or motion of the camera of the oral imaging device.

It should also be noted that, of course, if the machine-learning model is intended to determine the motion of the camera relative to the subject's mouth and is not provided data from an inertial measurement sensor, then it will need to be provided with at least two images of at least one oral feature of the subject (and the images' respective timestamps) such that motion between the at least two images can be predicted.

In addition, in some embodiments, the machine-learning model can learn from previous oral imaging sessions how the camera of the oral imaging device typically moves through a subject's mouth during an oral imaging session and thus use these learnings to fine-tune its future predictions of location and/or motion of said camera of the oral imaging device (including its predictions of future locations (i.e., based on present location and motion)).

The method 100 also comprises step 120 of controlling the oral imaging device to switch from a first imaging modality to a second imaging modality based on (i.e., responsive to) the determined location and/or motion.

In this embodiment, the second imaging modality (and the first imaging modality) comprises at least one of: white light; quantitative light-induced fluorescence; fluorescence imaging with reflectance enhancement; and infrared light. The first and second imaging modalities are always different from one another. For example, if the first imaging modality was white light, the second imaging modality could be quantitative light-induced fluorescence, fluorescence imaging with reflectance enhancement, or infrared light.

White light imaging modality can be understood as capturing an image while at least the portion of the subject's mouth in view of the camera is illuminated with white light. This is particularly useful for making a subject's teeth and gums visible.

Similarly, infrared imaging modality can be understood as capturing an image while at least the portion of the subject's mouth in view of the camera is illuminated with infrared light. This is particularly useful for visualizing cavities (i.e., caries).

Quantitative light-induced fluorescence (QLF) imaging modality can be understood as capturing an image while at least the portion of the subject's mouth in view of the camera is illuminated with blue light (e.g., light with a wavelength between 400 to 500nm, and preferably around 405nm). This results in plaque and tartar being more visible (than when using white light) through red self-fluorescence (while teeth are visible in green), though gums are not visible.

When QLF is being used, it is preferable to use a band pass filter in front of the camera to block blue excitation light, though this is not essential. In some embodiments, a band pass filter can be provided which is mechanically positionable, such that when the oral imaging device is in QLF imaging modality, the band pass filter can be automatically moved in front of the camera, and when the oral imaging device is in another imaging modality, the band pass filter can be moved away from the camera (i.e., out of view of the camera).

Fluorescence imaging with reflectance enhancement (FIRE) imaging modality can be understood as similar to QLF imaging, however, with a portion of white light also used to illuminate the portion of the subject's mouth in view of the camera (in addition to the blue light). This allows the gums to also be seen.

Of course, in other embodiments, as the skilled person would appreciate, the first and second imaging modalities can comprise any suitable imaging modalities for imaging the mouth of a subject and oral health features, such as, for example, polarized light or ultraviolet. Alternatively, or in addition, to using different lighting while capturing an image, two imaging modalities can also be different by virtue of different camera settings (e.g., a first image modality using white light with a first exposure/contrast setting and a second image modality also using white light but with a different second exposure/contrast setting). For instance, a camera setting which might be changed between different imaging modalities could comprise at least one of: field of view; shutter speed; aperture; exposure; contrast; and an intraoral scanner (IOS) distance spacer to mechanically force a specific distance between the camera and the subject of the image. In some embodiments, each imaging modality can include a lighting mode (e.g., white light, QLF, FIRE, or infrared) and an associated set of camera settings (e.g., field of view, shutter speed, aperture, etc.).

In some embodiments, step 120 can comprise controlling the oral imaging device further based on at least one demographic parameter of the subject. A demographic parameter can comprise, for example, at least one of: age; gender; race; ethnicity; weight; height; BMI; and geographic location. These demographic parameters can be used to (more accurately) estimate a 3D head model of the subject, and in this way, when combined with the determined location and/or motion of the camera, a more accurate estimation of when a stroke of the oral imaging device is at the back of the jaw (or the front) can be made (i.e., when a subject's scanning stroke has ended), and thus, for example, the oral imaging device can be controlled to switch imaging modality at a more effective time.

In this embodiment, the method 100 further comprises sequentially capturing an image of each of at least two different teeth of the subject respectively using the oral imaging device in the first modality. This can enhance the time efficiency of the method, ensuring that the oral imaging device is not needlessly switching imaging modality after imaging only one tooth in the first imaging modality. However, as would be clear to the skilled person, this is not essential to the functioning of the method 100 and in other embodiments, the oral imaging device may capture only one image in the first imaging modality before switching to the second imaging modality.

In an example of the invention, a first scan (comprising capturing at least one image of each of a plurality of teeth) can be performed using one set of camera and lighting settings (i.e., in a first imaging modality), with the oral imaging modality moving in a first direction (e.g., from the front of the mouth to the back of one side of the subject's jaw). Once it has been determined that the oral imaging device is at the back of the subject's mouth and/or has stopped moving (thus implying it has reached the back of the subject's mouth), the set of camera and lighting settings can be switched (i.e., can switch the oral imaging device to a second imaging modality) and then a second scan can be performed in the opposite direction (e.g., from the back of the mouth to the front). In this way, images can be captured of a subjects' teeth in multiple imaging modalities without them having to wait for the oral imaging device to switch imaging modality at each tooth.

In a specific example of the invention, using the ISO 3950 tooth enumeration system: a mouth section to-be-scanned is selected, in this case, top right outer; a subject starts imaging their mouth in a first imaging modality (e.g., white light) at frontal incisor number 21; the camera is moved to the next adjacent tooth in the segment, typically frontal incisor number 22; scanning adjacent teeth is repeated until the back the subject's mouth is reached, e.g., molar number 28; it is determined that the camera of the oral imaging device has reached the end of the segment (i.e., its stroke) via determination of location and/or motion (optionally using an AI to come to this determination); the oral imaging device is switched to a second imaging modality (e.g., QLF); the segment/stroke is performed but in the opposite direction (e.g., from molar number 28 to frontal incisor number 21); and the above procedure can then repeat for every mouth section the subject wishes to scan. In this way, instead of switching imaging modality (e.g., lighting) for each tooth, for example, progress through segments/strokes can automatically be detected and imaging modality can thus be switched less times while still obtaining images of the subjects' teeth in multiple imaging modalities.

A mouth section refers to any group/set of teeth and teeth surface (e.g., buccal, occlusal, distal, mesial, lingual). Example mouth sections can thus be the lingual frontal teeth, the left quadrant buccal or all the lower teeth. As described above, progress through scanning a mouth section can be determined in various ways, for example: the detection of no motion; the detection of change of motion direction; the detection of location (e.g., arriving at the back molar or beginning to image the same teeth again). The detection of movement direction and/or movement switching can be achieved with an (AI) algorithm based on camera images and/or an accelerometer or inertial measurement unit. Image registration techniques can also be used to detect movement and/or object detection and tracking methods.

Referring now to Fig. 2, there is depicted a diagram of a method 200 for controlling imaging modality of an oral imaging device comprising determining a motion of a camera according to a proposed embodiment.

Step 210 comprises determining a motion of the camera of the oral imaging device. This can be done in many ways, as the skilled person would understand. For example, determining a motion of the camera could be based on data from an inertial measurement sensor associated with (i.e., within or attached to) the oral imaging device. This provides an effective way to obtain the motion of the camera. In another example, determining a motion of the camera could be based on a captured series of images of at least one oral feature of the subject (for example, by using an object detector to detect (bounding box) and segment (mask) each individual tooth, such that by tracking the boundary boxes over the video frames, motion of the camera can be determined). As described above, an oral feature comprises a portion (i.e., part or whole) of at least one of: a tooth; gum; tongue; and a lip. Thus step 210 could be understood as being split into two steps: capturing a series of images of at least one tooth or the gum of the subject (using the camera of the oral imaging device); and analyzing the captured series of images to determine the motion of the camera. This would allow the motion of the camera to be obtained without the need for additional equipment (i.e., purely based on analysis of the distance moved by the camera between the images of the captured series of images). In yet another example, to enhance reliability and/or accuracy of the determined motion, the motion of the camera could be determined based on data from an inertial measurement sensor associated with the oral imaging device and a captured series of images of at least one oral feature of the subject.

In this embodiment, step 220 comprises controlling the oral imaging device to switch from a first imaging modality to a second imaging modality when the camera is determined to have stopped moving. For example, the camera typically stops moving when a user has reached the end of a stroke - this is thus a particularly beneficial time to switch imaging modality so that that the images of the same part of the subject's mouth can be captured in a new imaging modality on a backstroke.

Of course, in other embodiments, step 220 can comprise controlling the oral imaging device to switch imaging modalities based on the determined motion but not necessarily when the camera is determined to have stopped moving. For example, in another embodiment, step 220 may could comprise controlling the oral imaging device to switch imaging modalities when the camera is determined to be moving from one side of the subject's mouth to the other.

Referring now to Fig. 3, there is depicted a flow diagram of a method 300 for controlling imaging modality of an oral imaging device comprising determining a location of a camera according to a proposed embodiment. Step 320 is substantially the same as step 120 described in relation to method 100 of Fig. 1.

Step 310 comprises capturing an image of at least one oral feature of the subject using the camera of the oral imaging device. As described above, in some embodiments, the oral imaging device can comprise more than one camera, and in these embodiments, step 310 would be understood as capturing an image of at least one oral feature of the subject using at least one camera of the oral imaging device.

Step 315 comprises analysing the captured image to determine the location of the camera (at the time at which the image was captured, of course). This can be done in many ways, as the skilled person would understand. For example, the captured image could be analysed by a machine-learning model trained to determine the location of the camera based on an input image.

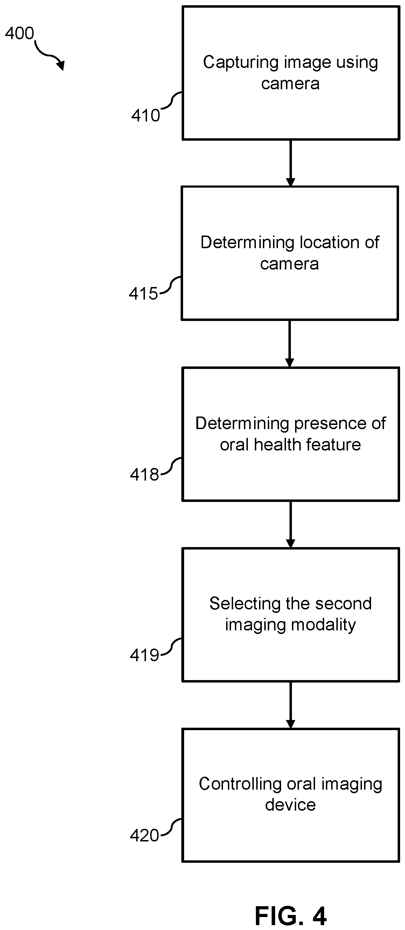

Referring now to Fig. 4, there is depicted a flow diagram of a method 400 for controlling imaging modality of an oral imaging device comprising predicting a future location of the camera according to a proposed embodiment. Steps 410 and 415 are substantially the same as steps 305 and 310 respectively as described in relation to method 300 of Fig. 3.

Step 418 comprises determining the presence of an oral health feature in view of the camera based on the determined location (determined in step 415) and oral health data describing the presence and location of at least one oral health feature in the mouth of the subject. For example, the oral health data could take the form of a database of oral health features known to be in the mouth of the subject (e.g., from prior imaging of the subject's mouth (e.g., using the same oral imaging device, essentially internally building up said oral health data) or dental records or any other suitable prior/historical information) and their respective locations. As the skilled person would understand, however, the oral health data need not take the form of a database and could take any suitable form for referencing. In this way, the presence of an oral health feature does not need to be directly determined by analysing an image (i.e., identifying the oral health feature itself in the image) but rather only the location of the camera needs to be determined and then prior/historical oral health data (e.g., describing that a subject's molar is known to have a filling) can be used to infer the presence of an oral health feature in view of the camera.

In some embodiments, the oral health data can also further describe the presence and location of at least one predicted oral health feature in the mouth of the subject. This could thus allow the oral imaging device to be controlled based on oral health features which are not 'known' to be present at a particular location in the subject's mouth but are predicted to be. For example, it may be known that if a subject has plaque on tooth 13, it is highly likely that they will also have plaque on tooth 14. The prediction of there being plaque present on tooth 14 can thus be used to control the oral imaging device (i.e., to select the imaging modality for imaging tooth 14). In this way, not only can prior knowledge be used to help determine when to switch imaging modality but also extrapolations of said prior knowledge. Predicting oral health features can also make use of demographic parameters to make more accurate predictions of the presence of oral health features, wherein a demographic parameter comprises at least one of: age; gender; race; ethnicity; weight; height; BMI; and geographic location. This can allow the presence of oral health features to be more accurately predicted, taking into account, for example, the probabilities of various oral health features being present depending on the subject's demographics. For instance, older subjects could be more likely to develop periodontitis, and subjects above a certain weight could be more likely to develop cavities. It should be noted that in other embodiments, the demographic parameter can comprise any suitable parameter which may influence the probabilities of various oral health features being present. In addition, in some embodiments, demographic parameters of a subject may be used to help estimate a 3D head model / jaw model of the subject which may then be used to help determine the presence of an oral health feature.

In this embodiment, an oral health feature comprises at least one of: a cavity; a crown; a bridge; a filling; plaque; tartar; gingivitis; periodontitis; tooth erosion; an abscessed tooth; malocclusion; a denture; a dental implant; braces; an impacted tooth; and a tumor. These are each useful oral health features to determine the presence of and thus allow a beneficial imaging modality for imaging said feature to be chosen. In other embodiments, however, an oral health feature can comprise any suitable type of oral health feature (i.e., any type of feature relevant to oral health).

It should be noted that in this embodiment, step 418 comprises not only determining the presence of an oral health feature but also identifying/determining the type of oral health feature, however, this is not essential to all embodiments which involve determining the presence of an oral health feature.

Step 419 comprises selecting the second imaging modality (i.e., the imaging modality being switched to) from a plurality of available imaging modalities (not including the first imaging modality) based on the type of oral health feature determined to be present. In this way, the most beneficial imaging modality for the type of oral health feature to-be-imaged can be switched to. It should be noted that in some embodiments, multiple imaging modalities may be determined to be equally beneficial (or beneficial in combination) for the same oral health feature, and thus in these cases, the second imaging modality can be selected to be one of these beneficial imaging modalities and then the method repeated with the other beneficial imaging modality(s) then selected as the second imaging modality (and so on). Images of the same oral health feature captured in multiple imaging modalities may increase the prediction accuracy of some specific types of oral health features.

Finally, step 420 comprises controlling the oral imaging device based on the determined presence of the oral health feature. For example, if a particular oral health feature is determined to be present in view of the camera, it could be preferred that the second imaging modality is used to image it, and thus the oral imaging device would be controlled to switch from a first imaging modality to the second. Of course, the oral imaging device could already be in the optimal available imaging modality for imaging the oral health feature and in this case, the oral imaging device would be controlled not to switch imaging modality but to stay in the imaging modality it is already in. In other words, step 420 could alternatively be understood as: controlling the imaging modality of the oral imaging device based on the determined presence of an oral health feature at the determined location, wherein the oral imaging device has at least two imaging modalities.

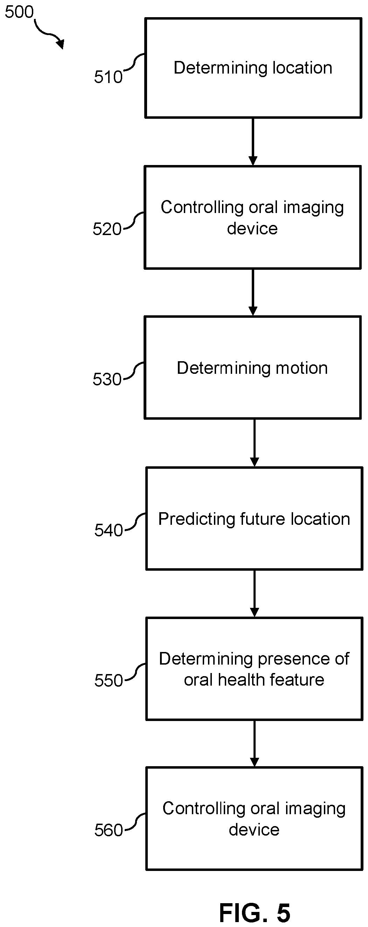

Referring now to Fig. 5, there is depicted a flow diagram of a method 500 for controlling imaging modality of an oral imaging device comprising predicting a future location of the camera according to a proposed embodiment.

Step 510 comprises determining a location of the camera of the oral imaging device (e.g., based on captured image) and thus is substantially the same as step 310 of method 300 of Fig. 3. In other embodiments, however, step 510 of determining a location of the camera can be performed in any suitable way, for example, a manual input at the beginning of an oral imaging procedure.

Step 520 comprises controlling the oral imaging device to switch from a first imaging modality to a second imaging modality based on the determined location, and is substantially the same as step 120 as described in relation to method 100 of Fig. 1. Step 530 comprises determining a motion of the camera and is substantially the same as step 210 of method 200 of Fig. 2. In other embodiments, step 530 could be replaced and the motion of the camera could be obtained in any other suitable way, e.g., without having to directly determine it, such as, for example, obtaining the motion of the camera from an external device/system.

Step 540 comprises predicting a future location of the camera at a future time point (i.e., time location/stamp) based on the determined motion of the camera (determined in step 530) and the determined location of the camera (determined in step 510).

Step 550 comprises determining the presence of an oral health feature at the predicted future location of the camera based on the predicted future location and oral health data describing the presence and location of at least one oral health feature in the mouth of the subject. The oral health data is substantially the same as the oral health data described in relation to step 418 of method 400 (and as described above, could also comprise the presence and location of at least one predicted oral health feature too).

Step 560 comprises controlling the oral imaging device to switch from a first imaging modality to a second imaging modality based on the determined presence of an oral health feature at the predicted future location of the camera. In this way, the oral imaging device can be further controlled based on what oral health feature will soon be in view of the camera.

For instance, in this embodiment, controlling the oral imaging device in step 560 specifically comprises controlling the oral imaging device to switch from a first imaging modality to a second imaging modality such that the oral imaging device will be in the second imaging modality at the future time point. In this way, use of the oral imaging device is made more efficient such that the oral imaging device can essentially pre-emptively switch imaging modality so that it is in the correct imaging modality by the time it reaches a location where it is known (or predicted) that a particular oral health feature is present. Of course, to ensure this is achieved, it must be known how long it takes for the oral imaging device to switch imaging modality (or the number of frames lost when switching modality) - this could be obtained as prior knowledge or, for example, learnt from prior oral imaging sessions. A user will then not have to wait once they reach said location for the oral imaging device to switch imaging modality.

In addition, in some embodiments, user guidance can be provided (e.g., via haptic feedback, audio instructions, visual guidance, etc.) based on the predicted future location of the camera such that the user is more likely to have the camera of the oral imaging device at the predicted future location at the future time point. In an extension of this concept, in some embodiments, an optimal path for the camera can also be predicted (personalised based on the user/subject and their oral health features) and the user guided along this path - the path being optimal in the sense that it will result in the fastest way to scan the user/subject's mouth while still capturing images of at least a portion of the subject's (most clinically relevant) oral health features in the optimal imaging modalities.

It should be noted that in some embodiments, the method 500 could comprise only steps 530 to 560, i.e., only switch imaging modality based on an oral health feature determined to be in view of the camera at a predicted future location of the camera (taking into account a determined location and motion), such that the oral imaging device has always switched imaging modality before the camera reaches said predicted future location. In other words, in some embodiments, the oral imaging device could be controlled to always pre-emptively switch in anticipation of a determined oral health feature being in view of the camera at a future predicted location.

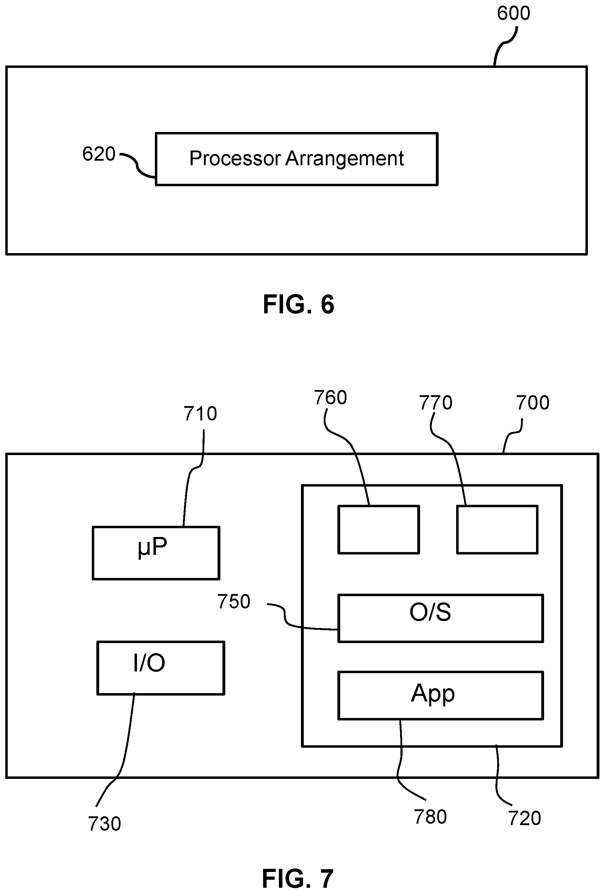

Referring now to Fig. 6, there is depicted a system 600 for controlling imaging modality of an oral imaging device according to a proposed embodiment. The system 600 comprises a processor arrangement 620.

The processor arrangement 620 is configured to perform any of the method disclosed herein (e.g., method 100, 200, 300, 400, or 500). For example, in this embodiment, the processor arrangement 620 is configured to perform method 100, i.e., to: determine a location and/or motion of a camera of the oral imaging device relative to a subject's mouth, wherein motion comprises at least one of: velocity; and acceleration; and control the oral imaging device to switch from a first imaging modality to a second imaging modality based on the determined location and/or motion.

In some embodiments, the system 600 can comprise part of the oral imaging device itself (the oral imaging device being a device capable of capturing an image in at least two imaging modalities and comprising at least one camera). In other embodiment, the system 600 can be external to the oral imaging device and communicate with it (e.g., obtain images captured by it and issue controlling instructions) either with wires or wirelessly.

Fig. 7 illustrates an example of a computer 700 within which one or more parts of an embodiment may be employed. Various operations discussed above may utilize the capabilities of the computer 700. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720 and one or more I/O devices 730 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 760, source code 770, and one or more applications 780 in accordance with exemplary embodiments. As illustrated, the application 780 comprises numerous functional components for implementing the features and operations of the exemplary embodiments. The application 780 of the computer 700 may represent various applications, computational units, logic, functional units, processes, operations, virtual entities, and/or modules in accordance with exemplary embodiments, but the application 780 is not meant to be a limitation.

The operating system 750 controls the execution of other computer programs, and provides scheduling, input-output control, file and data management, memory management, and communication control and related services. It is contemplated by the inventors that the application 780 for implementing exemplary embodiments may be applicable on all commercially available operating systems.

Application 780 may be a source program, executable program (object code), script, or any other entity comprising a set of instructions to be performed. When a source program, then the program is usually translated via a compiler (such as the compiler 760), assembler, interpreter, or the like, which may or may not be included within the memory 720, so as to operate properly in connection with the O/S 750. Furthermore, the application 780 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 730 may include input devices such as, for example but not limited to, a mouse, keyboard, scanner, microphone, camera, etc. Furthermore, the I/O devices 730 may also include output devices, for example but not limited to a printer, display, etc. Finally, the I/O devices 730 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 730 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at start-up, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 780 and the O/S 750 are read, in whole or in part, by the processor 710, perhaps buffered within the processor 710, and then executed.

When the application 780 is implemented in software it should be noted that the application 780 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 780 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 to 5, and the system of Fig. 6, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for controlling imaging modality of an oral imaging device, the method comprising:
determining a location and/or motion (110) of a camera of the oral imaging device relative to a subject's mouth, wherein motion comprises at least one of: velocity; and acceleration; and
controlling the oral imaging device (120) to switch from a first imaging modality to a second imaging modality based on the determined location and/or motion.

2. The computer-implemented method of claim 1, wherein determining a motion of the camera comprises determining the motion of the camera (210) based on data from an inertial measurement sensor associated with the oral imaging device.

3. The computer-implemented method of 1 or 2, wherein determining a motion of the camera comprises: capturing a series of images of at least one oral feature of the subject, wherein an oral feature comprises a portion of at least one of: a tooth; gum; tongue; and a lip; and analyzing the captured series of images to determine the motion of the camera.

4. The computer-implemented method of claims 2 or 3, wherein controlling the oral imaging device comprises controlling the oral imaging device to switch from a first imaging modality to a second imaging modality when the camera is determined to have stopped moving.

5. The computer-implemented method of claim 2 or 3, further comprising:
predicting a future location of the camera (540) at a future time point based on the determined motion of the camera and a determined present location of the camera; and
wherein controlling the oral imaging device comprises controlling the oral imaging device to switch from a first imaging modality to a second imaging modality such that the oral imaging device will be in the second imaging modality at the future time point.

6. The computer-implemented method of claim 5, further comprising:
determining the presence of an oral health feature (550) at the predicted future location of the camera based on the predicted future location and oral health data describing the presence and location of at least one oral health feature in the mouth of the subject; and
controlling the oral imaging device based on the determined presence of the oral health feature.

7. The computer-implemented method of any prior claim, wherein a location of the camera is determined, and wherein controlling the oral imaging device comprises:
determining the presence of an oral health feature (418) in view of the camera at the determined location based on the determined location and oral health data describing the presence and location of at least one oral health feature in the mouth of the subject; and
controlling the oral imaging device based on the determined presence of the oral health feature.

8. The computer-implemented method of claim 6 or 7, further comprising:
selecting the second imaging modality (419) from a plurality of available imaging modalities based on the type of oral health feature.

9. The computer-implemented method of any of claims 6 to 8, wherein an oral health feature comprises at least one of: a cavity; a crown; a bridge; a filling; plaque; tartar; gingivitis; periodontitis; tooth erosion; an abscessed tooth; malocclusion; a denture; a dental implant; braces; an impacted tooth; and a tumor.

10. The computer-implemented method of any of claims 6 to 9, wherein the oral health data further describes the presence and location of at least one predicted oral health feature in the mouth of the subject.

11. The computer-implemented method of any prior claim, wherein the second imaging modality comprises at least one of: white light; quantitative light-induced fluorescence; fluorescence imaging with reflectance enhancement; and infrared light.

12. The computer-implemented method of any prior claim, wherein determining a location of the camera comprises: capturing an image (305) of at least one oral feature of the subject, wherein an oral feature comprises a portion of at least one of: a tooth; gum; tongue; and a lip; and analyzing the captured image (310) to determine the location of the camera.

13. The computer-implemented method of any prior claim, wherein determining the location and/or motion of the camera comprises using a machine-learning model trained to determine the relative location and/or motion of the camera relative to the subject's mouth based on data from an inertial measurement sensor associated with the oral imaging device and/or at least one image of at least one oral feature of the subject, wherein an oral feature comprises a portion of at least one of: a tooth; gum; tongue; and a lip.

14. A computer program comprising code for implementing the method of any preceding claim when said program is run on a processor arrangement.

15. A system (600) for controlling imaging modality of an oral imaging device, comprising:
a processor arrangement (620) configured to:
determine a location and/or motion of a camera of the oral imaging device relative to a subject's mouth, wherein motion comprises at least one of: velocity; and acceleration; and
control the oral imaging device to switch from a first imaging modality to a second imaging modality based on the determined location and/or motion.
